# NEUE EUROPÄISCHE PATENTSCHRIFT

(11) **EP 2 111 125 B2**
(45) Veröffentlichungstag und Bekanntmachung des Hinweises auf die Entscheidung über den Einspruch: **16.11.2016**
(45) Hinweis auf die Patenterteilung: 16.03.2011
(21) Anmeldenummer: 08707899.4
(22) Anmeldetag: 15.01.2008
(51) Int. Cl.: A23L 33/105, A23K 20/179, A23K 20/163, A23L 5/44, A23P 10/30, A61K 31/07, A61K 8/34, A61K 8/35, A61K 8/73, A61Q 19/00, C09B 61/00

(54) **FLÜSSIGE FORMULIERUNGEN ENTHALTEND CAROTINOIDE**
LIQUID FORMULATIONS COMPRISING CAROTENOIDS
FORMULATIONS LIQUIDES COMPRENANT DES CAROTENOIDES

(30) Priorität: 16.01.2007 EP 07100633; 30.03.2007 EP 07105349
(43) Veröffentlichungstag der Anmeldung: 28.10.2009
(73) Patentinhaber: BASF SE, 67056 Ludwigshafen am Rhein (DE)
(72) Erfinder: KÖPSEL, Christian, 69469 Weinheim (DE); FELDTHUSEN JENSEN, Jesper, 55268 Nieder-Olm (DE)
(86) Internationale Anmeldenummer: PCT/EP2008/050401
(87) Internationale Veröffentlichungsnummer: WO 2008/087140

(56) Entgegenhaltungen:
- EP-A- 1 228 705
- EP-A1- 0 551 638
- EP-A1- 1 300 394
- WO-A1-03/017785
- WO-A1-2004/084862
- WO-A1-2005/060923
- WO-A1-2007/003543
- DE-A1- 3 119 383
- DE-A1- 4 200 728
- DE-A1-102004 046 026
- US-A- 3 110 598
- 'BASF Brochure "Products for the Dietary Supplement, Beverage and Food Industries-Technical Information', Mai 2005 Seiten 181 - 184
- BELITZ H.-D. ET AL: 'Lehrbuch der Lebensmittelchemie', Bd. 3, 1987, SPRINGER-VERLAG, BERLIN - HEIDELBERG - NEW YORK Seiten 1 - 6
- Research Disclosure 452 072
- Research Disclosure 170 064
- englische Übersetzung (=EP-A 1 782 803) von WO 2006/022187
- CADLE R.D.: 'Particle size determination', 1955, INTERSCIENCE PUBLISHERS, INC., NEW YORK Seiten 92 - 101
- KROSCHWITZ I.: 'Kirk-Othmer Encyclopedia of chemical technology', Bd. 22, 1997, JOHN WILEY & SONS, NEW YORK Seiten 256 - 257
- GENOVESE D.B. ET AL: 'Particle size determination of food suspensions: application to cloudy apple juice' JOURNAL OF FOOD PROCESS ENGINEERING Bd. 23, 2000, Seiten 437 - 452

## Beschreibung

Die vorliegende Erfindung betrifft flüssige Formulierungen, Verfahren zu ihrer Herstellung sowie ihre Verwendung als Zusatz zu Tierfuttermitteln und Lebensmitteln.

Die Formulierung von Carotinoiden stellt aufgrund ihrer Schwerlöslichkeit in Wasser und ihrer chemischen Instabilität eine besondere Herausforderung dar. Es gab daher zahlreiche Versuche, Carotinoidformulierungen bereitzustellen, die einerseits stabil sind und andererseits gute Bioverfügbarkeit aufweisen und bei der Anwendung die gewünschten Farbausbeuten liefern. Flüssige Carotinoidformulierungen sind von besonderem Interesse, weil die Gewinnung der Carotinoide in Pulverform und die Zubereitung einer flüssigen Formulierung durch den Anwender entfallen.

Die Herstellung von Carotinoid-Primärpartikeln mit einer Teilchengröße im Nanometerbereich ist entscheidend, um ausreichende Bioverfügbarkeit und Farbausbeuten zu erzielen. Um derartige Partikel herzustellen, hat man im Stand der Technik von Mahlverfahren Gebrauch gemacht, wie sie beispielsweise in der WO 91/06292 und WO 94/19411 beschrieben sind. Die Vermahlung der Carotinoide erfolgt mittels einer Kolloidmühle in wässrigen oder öligen Medien, wobei eine Partikelgröße im Nanometerbereich erzielt wird.

Die EP 1 460 060 A beschreibt die Herstellung von Luteinsuspensionen durch Vermahlen in einem Öl. Weiter beschreibt sie in kaltem Wasser dispergierbare Luteinformulierungen, die hergestellt werden durch Lösen von Lutein in einem mit Wasser nicht mischbaren organischen Lösungsmittel und Einkapseln in modifizierte Stärke in Anwesenheit eines Emulgators.

Die US 2005/0084462 beschreibt die Herstellung einer Farbstoffmischung zur Anwendung in Lebensmitteln, pharmazeutischen und kosmetischen Produkten durch Vermahlen eines Farbstoffs in Wasser in Anwesenheit von Gummi arabicum als Träger und anschließendes Erwärmen auf 30 bis 60 °C.

Die US 5,827,539 beschreibt das Vermahlen einer Dispersion eines Carotinoids in Öl, Bildung einer Emulsion mit einem Gemisch aus Stärke, Zucker und einem Antioxidans und abschließendes Sprühtrocknen.

Die WO 9613178 beschreibt die Herstellung stabiler Lycopin-Konzentrate durch Vermahlen des Lycopins in einem flüssigen Medium, in dem das Lycopin im Wesentlichen unlöslich ist. Als flüssiges Medium werden Glycerin, Propylenglykol oder Ethanol verwendet. Ein Hydrokolloid wird jedoch nicht eingesetzt.

Die WO 96/23429 beschreibt die Herstellung von öligen Astaxanthin-Suspensionen mit Partikelgrößen < 2 µm durch Vermahlen von Astaxanthin, wobei während oder nach dem Vermahlen ein Öl zugegeben wird. Die Anwendung von Schutzkolloiden oder Emulgatoren ist nicht offenbart. Es wird darauf hingewiesen, dass die Partikel zur Agglomeration neigen. Dementsprechend wird eine weitere Stabilisierung durch Aufbewahren der Suspension unter der Verfestigungstemperatur in Betracht gezogen.

Die WO 93/04598 beschreibt die Herstellung einer Carotinoidzusammensetzung, enthaltend ein Carotinoid in einem Öl, eine Dispersion eines wasserdispergierbaren Matrixbildners, beispielsweise ein Zucker, und eines Stabilisators, beispielsweise Gelatine oder Casein und einen Emulgator sowie gegebenenfalls ein nicht-öliges Lösungsmittel, wie Glycerin.

Die DE 10 2004 046026 beschreibt ein Verfahren zur Herstellung von Trockenpulvem eines Carotinoids, wobei man ein Carotinoid in eine wässrige molekulardisperse oder kolloiddisperse Lösung eines Gemisches aus Isomalt und eines Schutzkolloids dispergiert und die gebildete Dispersion durch Abtrennung des Wassers und ggf. zusätzlich verwendeter Lösungsmittel in ein Trockenpulver überführt.

Die DE 3119383 beschreibt ein Verfahren zur Herstellung von fein verteilten, pulverförmigen Carotinoidpräparaten, in denen das Carotinoid im Wesentlichen eine Teilchengröße von weniger als 0,5 µm besitzt, in dem man ein Carotinoid in einem flüchtigen, mit Wasser mischbaren organischen Lösungsmittel zwischen 50°C und 170°C innerhalb einer Zeit von weniger als 10 Sekunden löst, aus der erhaltenen molekulardispersen Lösung sofort durch schnelles Mischen mit einer wässrigen Lösung eines quellbaren Kolloids bei Temperaturen zwischen 0°C und 50°C das Carotinoid in kolloiddisperser Form ausfällt und die erhaltene Dispersion in an sich bekannter Weise von dem Lösungsmittel und dem Dispergiermedium befreit.

Die US-3,110,598 beschreibt ein Verfahren zur Herstellung einer ein fein verteiltes Carotinoid enthaltener Zubereitung, bei dem man eine Lösung eines Carotinoids in einem flüchtigen, wasserunlöslichen Lösungsmittel in eine ein quellbares Kolloid enthaltende wässrige Lösung emulgiert und das flüchtige Wasser unlösliche Lösungsmittel aus der Emulsion entfernt.

Die EP-A-1 228 705 beschreibt ein Verfahren zur Herstellung von Trockenpulvem eines Carotinoids durch Dispergieren eines Carotinoids in einer wässrigen molekulardispersen oder kolloiddispersen Lösung eines Gemisches aus Lactose und einem Schutzkolloid und Überführen der gebildeten Dispersion in ein Trockenpulver, wobei man als Schutzkolloid mindestens ein Sojaprotein verwendet.

Der vorliegenden Erfindung liegt die Aufgabe zugrunde, eine flüssige Carotinoid-Formulierung zur Verfügung zu stellen, die einfach herstellbar und vom Anwender ohne zusätzlichen Aufwand eingesetzt werden kann.

Überraschenderweise wurde nun gefunden, dass diese Aufgabe mit einer flüssigen Formulierung gelöst wird, die neben einem Carotinoid und einem hydrophilen Schutzkolloid wenigstens einen mit Wasser mischbaren Alkohol enthält.

Gegenstand der vorliegenden Erfindung ist daher eine flüssige Formulierung, umfassend wenigstens ein Carotinoid, ein hydrophiles Schutzkolloid in Form von Stärke-Octenylsuccinat, und Glycerin, wobei die mittlere Teilchengröße der Carotinoidkristalle und des hydrophilen Schutzkolloids < 5 µm beträgt.

Als Carotinoide können die bekannten, aus natürlichen Quellen oder synthetisch zugänglichen Vertreter eingesetzt werden. Dabei können die bekannten, aus natürlichen Quellen oder synthetisch zugänglichen Vertreter eingesetzt werden. Beispiele hierfür sind β-Carotin, Lycopin, Lutein, Astaxanthin, Zeaxanthin, Cryptoxanthin, Citranaxanthin, Canthaxanthin, Echinenon, Bixin, β-Apo-4-carotinal, β-Apo-8-carotinal, β-Apo-4-carotinsäureester, einzeln oder als Mischung. Bevorzugt sind Astaxanthin, β- Carotin, β-Apo-8-carotinal, β-Apo-8'-carotinsäureethylester, Canthaxanthin, Citranaxanthin, Echinenon, Lutein, Lycopin und Zeaxanthin. Besonders bevorzugt sind Astaxanthin und Canthaxanthin und insbesondere Astaxanthin.

Die Carotinoide weisen im allgemeinen eine Teilchengröße im Bereich von 50 nm bis 5 µm, vorzugsweise 100 nm bis 5 µm, besonders bevorzugt 100 nm bis 3 µm, 150 nm bis 2 µm und insbesondere 200 nm bis 1 µm, auf.

Als hydrophiles Schutzkolloid wird Stärke-Octenylsuccinat verwendet. Entsprechende Produkte sind im Handel unter der Bezeichnung Purity Gum 2000 von National Starch oder Clear Gum CO 01 von Roquette, Hi Cap 100 oder Capsul von National Starch erhältlich.

Die erfindungsgemäßen Formulierungen enthalten Glycerin.

Die Carotinoide sind in den erfindungsgemäßen Formulierungen im Allgemeinen in einer Menge im Bereich von 0,1 bis 50 Gew.-%, vorzugsweise 0,2 bis 30 Gew.-% und insbesondere 1,0 bis 15 Gew.%, bezogen auf das Gesamtgewicht der Formulierung, enthalten.

Die Menge an hydrophilem Schutzkolloid liegt im Allgemeinen im Bereich von 1 bis 40 Gew.-%, vorzugsweise 2 bis 30 Gew.-% und insbesondere 3 bis 25 Gew.-%.

Das Gewichtsverhältnis von Carotinoid zu hydrophilem Schutzkolloid liegt im Allgemeinen im Bereich von 5:1 bis 1:5, vorzugsweise 3:1 bis 1:3. Die Menge an Glycerin liegt im Allgemeinen im Bereich von 30 bis 95 Gew.%, insbesondere 30 bis 90 Gew.-% und besonders bevorzugt 35 bis 90 Gew.%, bezogen auf das Gesamtgewicht der Formulierung.

Die erfindungsgemäßen Formulierungen können Wasser in einer Menge bis zu 40 Gew.%, vorzugsweise bis zu 35 Gew.%, bezogen auf das Gesamtgewicht der Formulierung, enthalten.

Vorzugsweise enthalten die erfindungsgemäßen Formulierungen kein Öl.

Zur Erhöhung der Stabilität des Wirkstoffes gegen oxidativen Abbau ist es vorteilhaft, Stabilisatoren wie α-Tocopherol, t-Butylhydroxytoluol, t-Butylhydroxyanisol, Ascorbinsäure oder Ethoxyquin zuzusetzen.

Die erfindungsgemäßen Formulierungen können außerdem Hilfsstoffe, wie Verdicker, Hartfette, Chelatbildner, beispielsweise Alkali- oder Erdalkalisalze der Citronensäure, Phythinsäure oder Phosphorsäure und/oder Emulgatoren enthalten. Beispiele für Emulgatoren sind Ascorbylpalmitat, Polyglycerin-Fettsäureester, wie Polyglycerin-3-polyricinoleat (PGPR 90), Sorbitan-Fettsäureester, wie Sorbitanmonostearat (Span 60), PEG(20)-Sorbitolmonooleat, Propylenglykol-Fettsäureester oder Phospholipide, wie Lecithin.

Zur Herstellung der erfindungsgemäßen Formulierungen wird eine Dispersion des Carotinoids und des hxdrophilen Schutzkolloids in dem Glycerin vermahlen. Ein Teil des Glycerins kann jedoch auch während des Vermahlens zugegeben werden. Das Mahlen wird so lange durchgeführt, bis eine mittlere Teilchengröße der Carotinoidkristalle und des hydrophilen Schutzkolloids von < 5 µm, vorzugsweise < 1 µm, erreicht ist. Als Vorrichtung zum Vermahlen können übliche, dem Fachmann bekannte Vorrichtungen, beispielsweise Kolloidmühlen, Kugelmühlen, wie Dynomill Typ KDL oder Multilab eingesetzt werden.

Die erfindungsgemäßen Formulierungen können direkt wässrigen oder nicht wässrigen Zubereitungen zugesetzt werden. Vor der Zugabe können sie jedoch auch mit dem verwendeten Alkohol weiter verdünnt werden.

Die Formulierungen eignen sich unter anderem als Zusatzstoff zu Tierfuttermitteln und Lebensmittelzubereitungen bzw. Mischfutter, als Mittel für die Herstellung pharmazeutischer und kosmetischer Zubereitungen sowie für die Herstellung von Nahrungsergänzungspräparaten im Human- und Tierbereich. Bevorzugt lassen sich die Suspensionen als Futtermittelzusatz in der Tieremährung einsetzen, beispielsweise durch Einmischen in Futtermittelpellets bei der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als sogenannte "post-pelleting application". Vorzugsweise belädt man die Futtermittelpellets mit den Formulierungen unter vermindertem Druck.

Typische Einsatzgebiete im Lebensmittelbereich sind beispielsweise die Vitaminierung und Färbung von Getränken, Milchprodukten wie Joghurt, Milchmixgetränken oder Milchspeiseeis sowie von Puddingpulvern, Eiprodukten, Backmischungen und Süßwaren. Im Kosmetikbereich können die öligen Suspensionen beispielsweise für dekorative Körperpflegemittel, beispielsweise in Form einer Creme, einer Lotion, als Lippenstift oder Make-up, verwendet werden.

Gegenstand der Erfindung sind ferner Nahrungsergänzungsmittel, Tierfuttermittel, Lebensmittel sowie pharmazeutische und kosmetische Zubereitungen, welche die erfindungsgemäßen Formulierungen umfassen. Bevorzugt sind Tierfuttermittel, insbesondere Futtermittelpellets, die mit den Formulierungen beladen sind.

Unter Nahrungsergänzungspräparaten sowie pharmazeutischen Zubereitungen sind u.a. Tabletten, Dragees sowie bevorzugt Hart- und Weichgelatinekapseln zu verstehen, welche die erfindungsgemäßen Formulierungen umfassen.

Die nachfolgenden Beispiele erläutern die Erfindung, ohne sie zu begrenzen.

### Beispiel 1

### Herstellung einer 10,5 %igen β-Carotindispersion

In einem 2-I-Becherglas wurden 784,16 g Glycerin vorgelegt und 110,84 g (H₂O-Gehalt 5,27 %) Purity Gum 2000 (Octenylsuccinat-Stärke) eingerührt. Anschließend wurden 105,0 g kristallines β-Carotin eingerührt, bis es vollständig benetzt war (30 bis 40 min). Die so erhaltene Dispersion wurde anschließend mit einer Dynomill-Multilab unter Verwendung von SiLi Beads Zr-Kugeln mit Yttrium stabilisiert und mit einem Durchmesser von 0,4 bis 0,6 mm, der Firma Siegmund Lindner vermahlen. Der Mahlbehälter war mit 400 ml dieser Mahlkugeln befüllt. Das Vermahlen erfolgte bei einer Temperatur im Bereich von 54 bis 70 °C, einem Reibspalt von 0,10 mm und einer Drehzahl der Mühle von 2986 U min.

### Beispiel 2

### Herstellung einer 7,0 %igen β-Carotindispersion

Nach dem in Beispiel 1 beschriebenen Verfahren wurde eine Dispersion aus 210,0 g kristalinem β-Carotin, 221,68 g Purity Gum 2000 (H₂O-Gehalt 5,27 %) und 2568,32 g Glycerin vermahlen. Das Vermahlen erfolgte bei einer Temperatur im Bereich von 52 bis 68 °C. Nach 1,5 h wurde eine dünnflüssige Dispersion erhalten, in der das β-Carotin eine Teilchengröße von 438 nm hatte.

## Patentansprüche

1. Flüssige Formulierung, umfassend wenigstens ein Carotinoid, ein hydrophiles Schutzkolloid in Form von Stärke-Octenylsuccinat, und Glycerin, erhältlich durch Vermahlen einer Dispersion des Carotinoids und des hydrophilen Schutzkolloids in Glycerin bis eine mittlere Teilchengröße der Carotinoidkristalle und des hydrophilen Schutzkolloids < 5 µm erreicht ist.

2. Formulierungen nach Anspruch 1, die 0 bis 40 Gew.-% Wasser enthalten.

3. Formulierung nach Anspruch 1 oder 2, wobei es sich bei dem Carotinoid um Astaxanthin, β-Carotin, β-Apo-8-carotinal, β-Apo-8'-carotinsäureethylester, Canthaxanthin, Citranaxanthin, Echinenon, Lutein, Lycopin oder Zeaxanthin handelt.

4. Formulierungen nach einem der Ansprüche 1 bis 3, wobei es sich bei dem Carotinoid um Astaxanthin handelt.

5. Verwendung der Formulierung nach einem der Ansprüche 1 bis 4 als Zusatz zu Tierfuttermitteln, Lebensmitteln und Nahrungsergänzungsmitteln sowie pharmazeutischen und kosmetischen Mitteln.

6. Tierfuttermittel, Lebensmittel und Nahrungsergänzungsmittel, umfassend die Formulierung gemäß einem der Ansprüche 1 bis 4.

## Claims

1. A liquid formulation, comprising at least one carotenoid, a hydrophilic protective colloid in the form of starch octenyl succinate and glycerol, which formulation can be obtained by grinding a dispersion of the carotenoid and of the hydrophilic protective colloid in glycerol until a mean particle size of a carotenoid crystals and of the hydrophilic protective colloid of < 5 µm is achieved.

2. The formulations according to claim 1, which comprise from 0 to 40% by weight of water.

3. The formulation according to claim 1 or 2, wherein the carotenoid is astaxanthin, β-carotene, β-apo-8-carotenal, ethyl β-apo-8'-carotenate, canthaxanthin, citranaxanthin, echinenone, lutein, lycopene or zeaxanthin.

4. The formulations according to any of claims 1 to 3, wherein the carotenoid is astaxanthin.

5. The use of the formulation according to any of claims 1 to 4 as additive to animal feeds, foodstuffs and food supplements as well as pharmaceuticals and cosmetics.

6. An animal feed, foodstuff and food supplement, comprising the formulation according to any of claims 1 to 4.

## Revendications

1. Composition liquide, comprenant au moins un caroténoïde, un colloïde protecteur hydrophile sous forme d'octénylsuccinate d'amidon, et du glycérol, laquelle composition peut être obtenue par broyage d'une dispersion du caroténoïde et du colloïde protecteur hydrophile dans du glycérol jusqu'à obtenir une taille moyenne de particule des cristaux de caroténoïde et du colloïde protecteur hydrophile de < 5 µm.

2. Compositions selon la revendication 1, qui contiennent de 0 à 40 % en poids d'eau.

3. Composition selon la revendication 1 ou 2, dans laquelle le caroténoïde consiste en astaxanthine, β-carotène, β-apo-8-caroténal, β-apo-8'-caroténoate d'éthyle, canthaxanthine, citranaxanthine, échinénone, lutéine, lycopène ou zéaxanthine.

4. Compositions selon l'une quelconque des revendications 1 à 3, dans laquelle le caroténoïde est l'astaxanthine.

5. Utilisation de la composition selon l'une quelconque des revendications 1 à 4, comme additif à des aliments pour animaux, des produits alimentaires et des compléments nutritionnels ainsi qu'à des produits pharmaceutiques et cosmétiques.

6. Aliment pour animaux, produit alimentaire et complément nutritionnel, comprenant la composition selon l'une quelconque des revendications 1 à 4.
